# EUROPEAN PATENT APPLICATION

(11) **EP 4 389 171 A1**
(43) Date of publication of application: **26.06.2024**
(21) Application number: 22913235.2
(22) Date of filing: 29.06.2022
(51) Int. Cl.: A61M 5/158, A61M 5/142, A61M 5/168

(54) **BALANCED INDWELLING NEEDLE ASSEMBLY AND PASTING TYPE LIQUID MEDICINE INFUSION SYSTEM**

(30) Priority: 31.12.2021 CN 202111679352
(71) Applicant: Aaruy Medical Electronics Co., Ltd., Shenzhen, Guangdong 518000 (CN)
(72) Inventor: ZHOU, Qingxu, Shenzhen, Guangdong 518000 (CN)
(74) Representative: Petraz, Gilberto Luigi
(86) International application number: PCT/CN2022/102227
(87) International publication number: WO 2023/123946

(57) **Abstract**

Provided in the present application is a balanced indwelling needle assembly and a paste-type medicine infusion system, including a baseplate and a vertical indwelling needle provided in a central preset area of the baseplate; a side of the baseplate is an applying surface and an opposite side of the baseplate is a mounting surface; the vertical indwelling needle is formed with an indwelling needle catheter extending vertically away from the applying surface; the applying surface is formed with an adhesive layer for being fixed onto a surface of a patient's skin; the vertical indwelling needle is formed with a fluid inlet connector on the mounting surface, the fluid inlet connector being in communication with a top inlet of the indwelling needle tube; the mounting surface is formed with a mounting part; the mounting part is used to detachably fix a main engine unit of a medicine infusion system on the mounting surface; and when the main engine unit is mounted and fixed on the mounting surface, the fluid inlet connector is in sealed communication with a medicine delivery connector of a medicine delivery device of the main engine unit. The indwelling needle catheter for indwelling into the skin of the balanced indwelling needle assembly is relatively slightly affected by the displacement of the main engine unit, which effectively reduces the risk of increasing the patient's skin wound due to the patient's movement.

## Description

### Field of the invention

The present application relates to the technical field of medical instruments, particularly a balanced indwelling needle assembly and a paste-type medicine infusion system.

### Background of the invention

Insulin injection has become one of the core means of modern comprehensive treatment solutions for diabetes. Insulin pumps may simulate the normal pancreas to continuously and precisely inject insulin into the body at different speeds. Insulin pumps simulate the working mechanism of the human pancreas by presetting the amount of injected insulin to ensure the patient has a normal glucose level.

The solutions of the prior art are divided into two types, i.e., external type and paste type: the external type insulin pump, with large volume and exposed cannula, requires to be worn on the belt or related accessories, which is inconvenient to be used and is prone to lead to a series of safety problems, such as catheter entanglement and clogging; the current paste-type insulin pumps on the market, although reduced in size, are limited by the layout of the structure itself, and the indwelling needle is commonly provided at an end of the overall structure. As the human body moves, the whole device is driven up and down like a seesaw, and the indwelling needle is just at the front of the seesaw, which is the position with the largest movement stroke in the whole device. With irregular movement, it is difficult to avoid increasing the patient's skin wound. Moreover, when the pump is impacted or scratched, the wound on the patient's skin is even more serious. Also, besides the above problems with the paste-type insulin pumps, similar problems exist in some other paste-type medicine delivery devices that require precise control of the infusion amount.

In summary, how to solve the problem of paste-type medicine delivery device easily enlarging patient's skin wounds during use has become a technical challenge that those skilled in the art urgently need to address.

### Summary of the invention

In view of the above, provided in the present application is a balanced indwelling needle assembly and a paste-type medicine infusion system, so as to solve the problem that the paste-type medicine delivery device is prone to enlarge the patient's skin wound during use.

In order to achieve the objective mentioned above, provided in the present application is a balanced indwelling needle assembly, including a baseplate and a vertical indwelling needle provided on a central preset area of the baseplate; a side of the baseplate is an applying surface and an opposite side of the baseplate is a mounting surface;

the vertical indwelling needle is formed with an indwelling needle tube extending vertically away from the applying surface; the applying surface is formed with an adhesive layer for being fixed onto a surface of a patient's skin;

the vertical indwelling needle is formed with a fluid inlet connector on the mounting surface, the fluid inlet connector being in communication with a top inlet of the indwelling needle tube; the mounting surface is formed with a mounting part; the mounting part is used to detachably fix a main engine unit of a medicine infusion system on the mounting surface; and when the main engine unit is mounted and fixed on the mounting surface, the fluid inlet connector is in sealed communication with a medicine delivery connector of a medicine delivery device of the main engine unit.

Optionally, the mounting part is a plurality of first buckles formed around the mounting surface, and the main engine unit is provided with a plurality of second buckles correspondingly snap-fitted with the first buckles.

Optionally, any one of the first buckle and the second buckle is an elastic snap latch, and the other is a snap-fit slot.

Compared to the description of the background of the present application, the balanced indwelling needle assembly mentioned above includes a baseplate and a vertical indwelling needle provided on a central preset area of the baseplate; a side of the baseplate is an applying surface and an opposite side of the baseplate is a mounting surface; the vertical indwelling needle is formed with an indwelling needle tube extending vertically away from the applying surface; the applying surface is formed with an adhesive layer for being fixed onto a surface of a patient's skin; the vertical indwelling needle is formed with a fluid inlet connector on the mounting surface, the fluid inlet connector being in communication with a top inlet of the indwelling needle tube; the mounting surface is formed with a mounting part; the mounting part is used to detachably fix a main engine unit of a medicine infusion system on the mounting surface; and when the main engine unit is mounted and fixed on the mounting surface, the fluid inlet connector is in sealed communication with the medicine delivery connector of the medicine delivery device of the main engine unit. During the practical application of the balanced indwelling needle assembly, the main engine unit of the medicine infusion system may be fixed to the mounting surface of the baseplate by the mounting part. Furthermore, after the installation is completed, the fluid inlet connector on the mounting surface and the medicine delivery connector of the medicine delivery device of the main engine unit may be in sealed communication. The connecting tube between them is omitted, with higher degree of integration, smaller volume and lighter weight, which may then be fixed to the patient's skin surface through the adhesive layer of the applying surface of the baseplate. For a patient using the balanced indwelling needle assembly mentioned above, since the vertical indwelling needle is provided in the central preset area of the baseplate, the indwelling needle catheter for indwelling into the skin is relatively slightly affected by the displacement of the main engine unit, which effectively reduces the risk of increasing the patient's skin wound due to the patient's movement.

Additionally, provided in the present application is also a paste-type medicine infusion system, including a balanced indwelling needle assembly described in any one of the solutions mentioned above and a main engine unit. Since the balanced indwelling needle assembly provides the technical effects mentioned above, a paste-type medicine infusion system provided with the balanced indwelling needle assembly should also provide the corresponding technical effects, which is not repeated herein.

Optionally, the main engine unit includes a medicine delivery device and a controlling device for controlling a medicine dispensing amount of the medicine delivery device; the medicine delivery device is integrated into a first shell, and the controlling device is integrated into a second shell; the first shell is formed with an insertion slot, and the second shell is formed with an insertion part inserted to fit into the insertion slot; a direction of insertion fit between the insertion part and the insertion slot is parallel to the mounting surface of the baseplate of the balanced indwelling needle assembly; and when the insertion part is inserted into the insertion slot, the controlling device controls the medicine dispensing amount of the medicine delivery device.

Optionally, the medicine delivery device is provided with a piston for adjusting the medicine dispensing amount; the piston is provided in the insertion slot; the controlling device is provided with a drive pusher; the drive pusher is provided on the insertion part; and when the insertion part is inserted into the insertion slot, the drive pusher drives the piston to move and to adjust the medicine dispensing amount.

Optionally, the insertion slot and the insertion part are fixed by a snap fit.

Optionally, the insertion part is formed with an avoiding groove for avoiding the medicine delivery connector of the medicine delivery device.

Optionally, the second shell includes a top cover with a bottom opening and a bottom cover covering the bottom opening of the top cover, and an accommodating chamber is formed within the top cover to contain the controlling member of the controlling device.

Optionally, a side of the first shell away from the insertion slot is also provided with a battery mounting groove.

### Brief description of the drawings

In order to illustrate the technical solutions of the embodiments of the present application or of the prior art more clearly, the following drawings are briefly described as required in the context of the embodiments or the prior art. Obviously, the following drawings illustrate only some of the embodiments of the present application. Other relevant drawings may be obtained on the basis of the provided drawings without any creative effort by those skilled in the art.
Fig. 1 is a structural diagram in a front view of the vertical indwelling needle provided in an embodiment of the present application;
Fig. 2 is a structural diagram in a top view of the vertical indwelling needle provided in an embodiment of the present application;
Fig. 3 is a structural diagram in a left view of the vertical indwelling needle provided in an embodiment of the present application;
Fig. 4 is a structural diagram in a front view of the paste-type medicine infusion system provided in an embodiment of the present application;
Fig. 5 is a structural diagram in a left view of the paste-type medicine infusion system provided in an embodiment of the present application;
Fig. 6 is a structural diagram in a front view of the controlling device provided in an embodiment of the present application;
Fig. 7 is a structural diagram in a front view of the medicine delivery device provided in an embodiment of the present application;
Fig. 8 is a structural diagram in a bottom view of the medicine delivery device provided in an embodiment of the present application;
Fig. 9 is a structural diagram in a back view of the medicine delivery device provided in an embodiment of the present application.

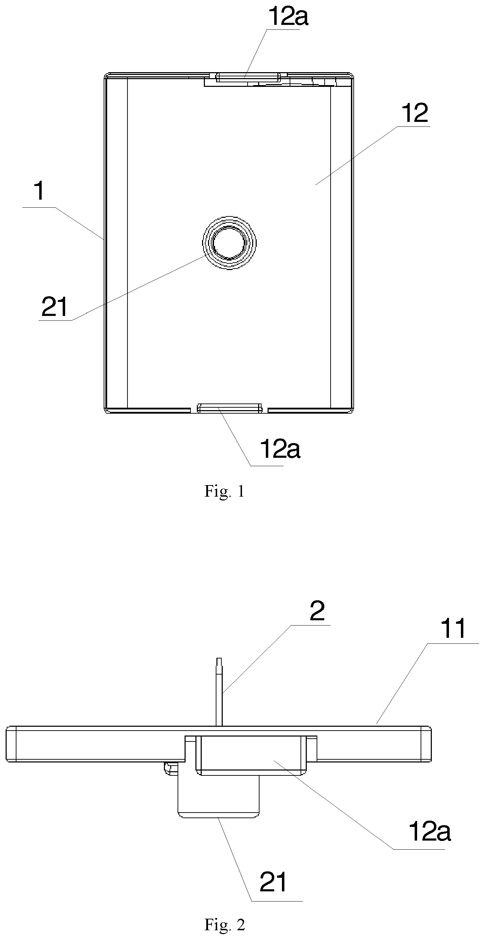

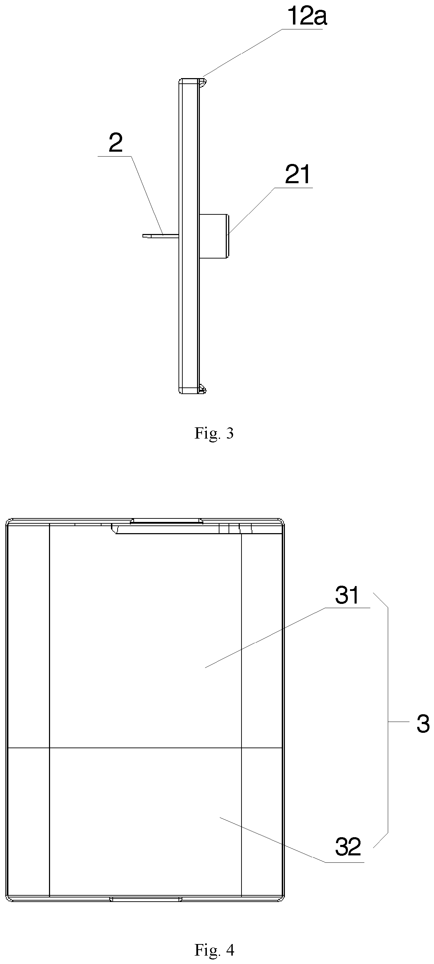

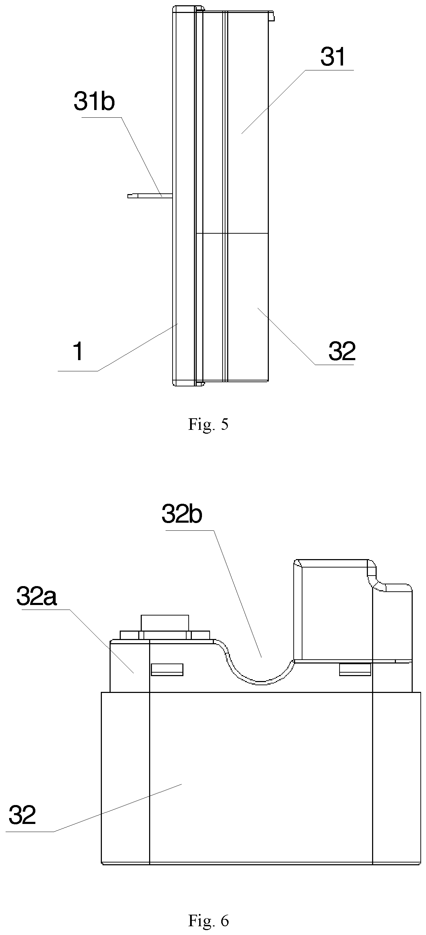

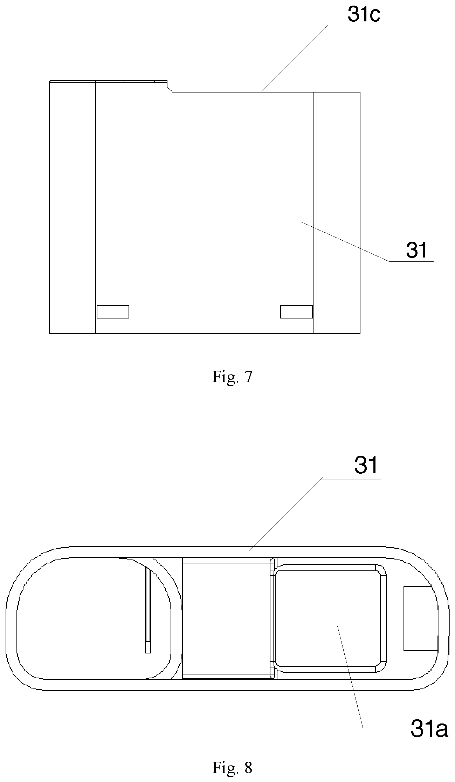

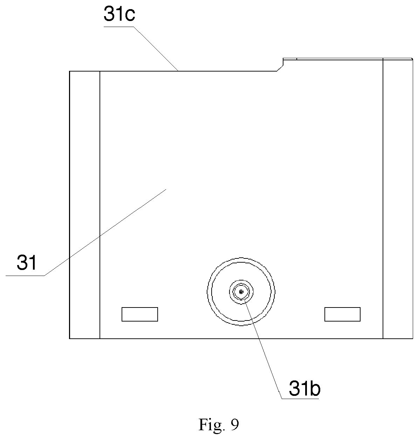

Labels in figures 1-9 are:
Baseplate 1; applying surface 11; mounting surface 12; mounting part 12a; vertical indwelling needle 2; fluid inlet connector 21; main engine unit 3; medicine delivery device 31; insertion slot 31a; medicine delivery connector 31b; battery mounting slot 31c; controlling device 32; insertion part 32a; avoiding groove 32b.

### Detailed description of the preferred embodiments

The core of the present application is to provide a balanced indwelling needle assembly and a paste-type medicine infusion system, so as to solve the problem that the paste-type medicine delivery device is prone to increase the patient's skin wound during use.

For a better understanding of the provided solutions in the present application by those skilled in the art, the technical solutions in the examples of the present application are clearly and completely described and discussed below in conjunction with the attached drawings of the embodiments of the present application. Obviously, the examples described herein are only some of the examples of the present application but not all of them. Based on the embodiments in the present application, all other embodiments obtained by those skilled in the art without creative efforts fall within the scope of protection of the present application.

As shown in Fig. 1 to Fig. 9, provided in the embodiment of the present application is a balanced indwelling needle assembly, including a baseplate 1 and a vertical indwelling needle 2 provided in a central preset area of the baseplate 1; a side of the baseplate 1 is an applying surface 11 and an opposite side of the baseplate 1 is a mounting surface 12; the vertical indwelling needle 2 is formed with an indwelling needle catheter extending vertically away from the applying surface 11; the applying surface 11 is formed with an adhesive layer for being fixed onto a surface of a patient's skin; the vertical indwelling needle 2 is formed with a fluid inlet connector 21 on the mounting surface 12, the fluid inlet connector 21 being in communication with a top inlet of the indwelling needle tube; the mounting surface 12 is formed with a mounting part 12a; the mounting part 12a is used to detachably fix a main engine unit 3 of a medicine infusion system on the mounting surface 12; and when the main engine unit 3 is mounted and fixed on the mounting surface 12, the fluid inlet connector 21 is in sealed communication with a medicine delivery connector 31b of a medicine delivery device 31 of the main engine unit 3.

During the practical application of the balanced indwelling needle assembly, the main engine unit of the medicine infusion system may be fixed to the mounting surface of the baseplate by the mounting part. Furthermore, after the installation is completed, the fluid inlet connector on the mounting surface and the medicine delivery connector of the medicine delivery device of the main engine unit may be in sealed communication. The connecting tube between them is omitted, with a higher degree of integration, smaller volume and lighter weight, which may then be fixed to the patient's skin surface through the adhesive layer of the applying surface of the baseplate. For a patient using the balanced indwelling needle assembly mentioned above, since the vertical indwelling needle is provided in the central preset area of the baseplate, the indwelling needle catheter for indwelling into the skin is relatively slightly affected by the displacement of the main engine unit, which effectively reduces the risk of increasing the patient's skin wound due to the patient's movement.

In some specific implementations, the mounting part 12a may be specifically a plurality of first buckles formed around the mounting surface 12, and the main engine unit 3 is provided with a plurality of second buckles correspondingly snap-fitted with the first buckles. By designing the mounting part 12a as a snap-fit structure to fit with the main engine unit 3, the disassembly and assembly of them become much easier. It is to be understood that the connection means of adopting a snap-fit mentioned above is only a preferred embodiment of the present application, and other detachable methods commonly used by those skilled in the art may be adopted in the practical applications. For example, by means of fasteners for fixing, only compared to the snap-fit connection, it is relatively inconvenient to disassemble and assemble, and it may be selected according to the actual demand in the practical applications, which is not limited more specifically herein.

It is to be noted that the specific structure of the snap-fit between the first buckle and the second buckle mentioned above may be that any one of the first buckle and the second buckle is an elastic snap latch, and the other is a snap-fit slot. In practical applications, the configuration may be selected according to the actual demand.

Additionally, provided in the present application is also a paste-type medicine infusion system, including a balanced indwelling needle assembly described by the solutions mentioned above and a main engine unit. Since the balanced indwelling needle assembly provides the technical effects mentioned above, a paste-type medicine infusion system provided with the balanced indwelling needle assembly should also provide the corresponding technical effects, which is not repeated herein.

In some more specific implementations, the main engine unit 3 mentioned above may specifically include a medicine delivery device 31 and a controlling device 32 for controlling a medicine dispensing amount of the medicine delivery device 31, in which the medicine delivery device 31 is integrated into a first shell, and the controlling device 32 is integrated into a second shell; the first shell is formed with an insertion slot 31a, and the second shell is formed with an insertion part 32a inserted to fit into the insertion slot 31a; a direction of insertion fit between the insertion part 32a and the insertion slot 31a is parallel to the mounting surface 12 of the baseplate 1 of the balanced indwelling needle assembly; and when the insertion part 32a is inserted into the insertion slot 31a, the controlling device 32 controls the medicine dispensing amount of the medicine delivery device 31. By integrating the medicine delivery device 31 into the first shell and the controlling device 32 into the second shell, and by means of the insertion structure between the insertion slot and the insertion part, it is more convenient to connect and fix them. It is much easier to achieve that the main engine unit is fixed to the mounting surface of the baseplate through the mounting part, with a higher degree of integration, which contributes to reducing the volume of the entire unit and provides a more aesthetically pleasing appearance.

In the further implementation, the medicine delivery device 31 mentioned above may be provided with a piston for adjusting the medicine dispensing amount; the piston is provided in the insertion slot 31a; the controlling device 32 is provided with a drive pusher; the drive pusher is provided on the insertion part 32a; and when the insertion part 32a is inserted into the insertion slot 31a, the drive pusher drives the piston to move and to adjust the medicine dispensing amount. It should be noted that the controlling member of the controlling device 32, besides providing with a drive pusher, generally includes a battery, a drive main engine, and other devices to cooperate with the drive pusher to accomplish corresponding actions. It is to be understood that the means of adjusting the medicine dispensing amount by pushing the piston through the drive pusher is only a preferred embodiment of the present application, and other drive methods may be adopted in the practical applications, which is not limited more specifically herein.

In some more specific implementations, the insertion slot 31a and the insertion part 32a may be fixed by a snap-fit after completing the insertion fit therebetween, or it may be fixed by other detachable fixing methods commonly used by those skilled in the art, which is not limited more specifically herein.

In some specific implementations, the insertion part 32a mentioned above is generally further formed with an avoiding groove 32b for avoiding the medicine delivery connector 31b of the medicine delivery device 31. By designing an avoidance groove, it may avoid interference with medicine delivery connector after the insertion part is inserted into the insertion slot within a limited insertion fit space.

In some specific implementations, the second shell mentioned above may specifically include a top cover with a bottom opening and a bottom cover covering the bottom opening of the top cover, and an accommodating chamber is formed within the top cover for containing the controlling member of the controlling device 32.

Additionally, it is to be noted that a side of the first shell away from the insertion slot 31a may also be provided with a battery mounting groove 31c.

It should be noted that the various embodiments in the present specification are described in a progressive manner. Each embodiment focuses on the differences with other embodiments. It is sufficient to refer to each embodiment for the same and similar parts.

Additionally, in the description of the present application, it is to be noted that the related terms "central", "up", "down", "left", "right", "vertical", "horizontal", "inside", "outside", if any, and other orientation or position relationships are based on the orientation or position relationships shown in the attached drawings. It is only intended to facilitate description of the present application and simplify description, but not to indicate or imply that the referred device or element has a specific orientation, or is constructed and operated in a specific orientation. Therefore, they should not be construed as a limitation of the present application. Furthermore, the related terms "first", "second", "third", if any, are used for descriptive purposes only and are not to be understood as indicating or implying relative importance.

It should also be noted that, as used herein, the related terms such as "include", "comprise", or any other derivatives and conjugations thereof, are intended to cover nonexclusive inclusion. Thus, an object or apparatus including a series of elements includes not only those elements, but also other elements that are not explicitly listed, or that are inherent to such an object or apparatus. In the absence of further limitation, an element defined by the phrase "including a/an ..." does not exclude the existence of another identical element in the object or apparatus including the aforementioned element.

Specific embodiments are used herein to illustrate the principles and implementations of the present application, and the above illustrations of the embodiments are only used to assist in the understanding of the core principles of the present application. It should be noted that for those skilled in the art, a plurality of improvements and modifications may be made without departing from the principles of the present application, which also fall within the scope of protection of the claims of the present application.

## Claims

1. A balanced indwelling needle assembly, comprising a baseplate (1) and a vertical indwelling needle (2) provided in a central preset area of the baseplate (1); a side of the baseplate (1) is an applying surface (11) and an opposite side of the baseplate (1) is a mounting surface (12);
the vertical indwelling needle (2) is formed with an indwelling needle catheter extending vertically away from the applying surface (11); the applying surface (11) is formed with an adhesive layer for being fixed onto a surface of a patient's skin;
the vertical indwelling needle (2) is formed with a fluid inlet connector (21) on the mounting surface (12), the fluid inlet connector (21) being in communication with a top inlet of an indwelling needle tube; the mounting surface (12) is formed with a mounting part (12a); the mounting part (12a) is used to detachably fix a main engine unit (3) of a medicine infusion system on the mounting surface (12); and when the main engine unit (3) is mounted and fixed on the mounting surface (12), the fluid inlet connector (21) is in sealed communication with a medicine delivery connector (31b) of a medicine delivery device (31) of the main engine unit (3).

2. The balanced indwelling needle assembly according to claim 1, wherein the mounting part (12a) is a plurality of first buckles formed around the mounting surface (12), and the main engine unit (3) is provided with a plurality of second buckles correspondingly snap-fitted with the first buckles.

3. The balanced indwelling needle assembly according to claim 1, wherein any one of the first buckle and the second buckle is an elastic snap latch, and the other is a snap-fit slot.

4. A paste-type medicine infusion system, comprising a balanced indwelling needle assembly and a main engine unit, wherein the balanced indwelling needle assembly is the balanced indwelling needle assembly as claimed in any one of claims 1-3.

5. The paste-type medicine infusion system according to claim 4, wherein the main engine unit (3) comprises a medicine delivery device (31) and a controlling device (32) for controlling a medicine dispensing amount of the medicine delivery device (31); the medicine delivery device (31) is integrated into a first shell, and the controlling device (32) is integrated into a second shell; the first shell is formed with an insertion slot (31a), and the second shell is formed with an insertion part (32a) inserted to fit into the insertion slot (31a); a direction of insertion fit between the insertion part (32a) and the insertion slot (31a) is parallel to the mounting surface (12) of the baseplate (1) of the balanced indwelling needle assembly; and when the insertion part (32a) is inserted into the insertion slot (31a), the controlling device (32) controls the medicine dispensing amount of the medicine delivery device (31).

6. The paste-type medicine infusion system according to claim 5, wherein the medicine delivery device (31) is provided with a piston for adjusting the medicine dispensing amount; the piston is provided in the insertion slot (31a); the controlling device (32) is provided with a drive pusher; the drive pusher is provided on the insertion part (32a); and when the insertion part (32a) is inserted into the insertion slot (31a), the drive pusher drives the piston to move and to adjust the medicine dispensing amount.

7. The paste-type medicine infusion system according to claim 5, wherein the insertion slot (31a) and the insertion part (32a) are fixed by a snap fit.

8. The paste-type medicine infusion system according to claim 5, wherein the insertion part (32a) is formed with an avoiding groove (32b) for avoiding the medicine delivery connector (31b) of the medicine delivery device (31).

9. The paste-type medicine infusion system according to claim 5, wherein the second shell comprises a top cover with a bottom opening and a bottom cover covering the bottom opening of the top cover, and an accommodating chamber is formed within the top cover for containing a controlling member of the controlling device (32).

10. The paste-type medicine infusion system according to claim 5, wherein a side of the first shell away from the insertion slot (31a) is also provided with a battery mounting groove (31c).
